# EUROPEAN PATENT APPLICATION

(11) **EP 1 362 911 A1**
(43) Date of publication of application: **19.11.2003**
(21) Application number: 02700673.3
(22) Date of filing: 21.02.2002
(51) Int. Cl.: C12N 15/09, C12N 1/15, C12N 1/19, C12N 1/21

(54) **TRANSGENIC ANIMAL HAVING DRUG METABOLISM ENZYME GENE AND UTILIZATION THEREOF**

(30) Priority: 23.02.2001 JP 2001047735
(71) Applicant: Gencom Corporation, Machida-shi, Tokyo 194-8511 (JP)
(72) Inventor: KATSUKI, Motoya, Minato-ku, Tokyo 106-0032 (JP); KAMATAKI, Tetsuya, Sapporo-shi, Hokkaido 064-0808 (JP); TERANISHI, Yutaka, Sagamihara-shi, Kanagawa 229-0021 (JP); ISHIDA, Mitsuyoshi, Shinagawa-ku, Tokyo 140-0011 (JP); KATO, Minoru, Setagaya-ku, Tokyo 158-0084 (JP)
(74) Representative: Sternagel, Fleischer, Godemeyer & Partner Patentanwälte
(86) International application number: JP0201555
(87) International publication number: WO02066635

(57) **Abstract**

An object of the present invention is to provide a transgenic animal which is useful in a test for forecasting fetal toxicity and teratogenicity of drugs. The present invention provides a transgenic non-human animal or a part thereof wherein several foreign (human-type) proteins which are involved in the drug metabolism or mutant proteins thereof are expressed in the body; a recombinant gene which is useful for the production of said non-human animal; and a method for screening a substance having fetal toxicity and teratogenicity using said non-human animal.

## Description

### Technical Field

The present invention relates to a recombinant gene containing a human drug metabolizing enzyme gene, a transgenic non-human animal into which several recombinant genes each containing human drug metabolizing enzyme have been introduced, and the use thereof.

### Background Art

In the development of a new drug, reproduction tests including a test on teratogeniciₜy must be carried out as one of the toxicity tests in the preclinical trials. Before the Thalidomide Case, the effect of a drug on a fetus had hardly been examined. After the Thalidomide Case in 1961, "Animal experimentation guidelines concerning an effect of a drug on fetus" was issued in 1963 in Japan. In 1975, it was amended to "Animal experimentation guidelines concerning an effect of a drug on reproduction," and in accordance with the FDA system of the U.S.A., experimentation was separated into three segments of Segment 1, Segment 2, and Segment 3.

Segment 2 is referred to as the teratogenicity test, which is carried out by administering a test drug to at least two types of animals including nonrodents at the stage of organ formation, and inspecting the teratogenesis of the fetus. However, there is always a limit due to differences among species in the result of animal experimentation. If this forecast system did not function satisfactorily, the results could always be disastrous. A classical example of failure in this forecast method is the Thalidomide Case, and in this incidence, the fetus did not develop any malformation in the early test using a rat (Somers, G. F. et al., Pharmacological properties of thalidomide, a new sedative hypnotic drug, Br. J. PharmacoL, 15, 111-116, 1960).

Most of the teratogenicity tests are carried out using mice or rabbits, and particularly rats. As described above, however, higher primates such as Rhesus monkeys are more appropriate models than rodents and rabbits in order to forecast the effects of teratogenicity. Direct comparison with known teratogen is reported by Wilson and Frandkin, "Comparison of teratogenic sensitivity of rhesus monkeys," Teratology, 2, 272, (1960) and Wilson, "Use of rhesus monkeys in teratological studies", Fed. Proc. 30, (1) 104-109, (1970). In order to make progress in such research, drug administration was attempted at corresponding generation stages. When acetazolamide, acetylsalicylic acid, retinoic acid, methotrexate, 5-fluorouracil, hydroxylurea, or vincristine was administered to rats, teratogenicity was observed at a lower dose as compare with the case of Rhesus monkeys. In contrast, the sensitivity of rats to thalidomide was significantly lower than that of Rhesus monkeys. However, significant difference was observed in the sensitivity between these two species in a quantitative sense, and this variation depends on the type *of* substance administered. The sensitivity of rats to methotrexate was higher than that of monkeys by 20 times, but the difference was 10 times or less in the remaining 6 types of drugs. On the other hand, the sensitivity of monkeys to thalidomide was 10 times or more than that of rats.

P450 is widely distributed in the organism world, and its various functions have been revealed. The primary structures of 500 types or more molecular species of P450 have been revealed, and they are classified into families and subfamilies based on sequence homologies. The classification is well matched with physiological roles. Among currently identified 65 families, 14 families are classified into the mammalian species. Among them, 4 families are associated with drug metabolism; 8 families are on the synthesis path for cholesterol, steroid hormone and bile acid, and 2 families correspond to a certain type of prostaglandin synthetase.

About 80% of drug metabolism is said to be associated with the P450 enzyme group, which constitutes a large gene superfamily. Since P450 metabolizes xenobiotic substances such as drugs and toxic agents, extensive research on P450 has been conducted in association with efficacy of drugs and toxicity of medicinal toxicants. P450 which is involved in the metabolism of xenobiotic substances such as drugs, is substantially limited to the molecular species belonging to the CYP1-CYP3 families, and some molecular species of CYP4 are unusually involved in the metabolism of xenobiotic substances. The CYP2 group comprises many subfamilies, and their properties are somewhat different depending on the type of the subfamily. Moreover, molecular species corresponding to animal species are not always available. CYP2E1 is induced by alcohol and the like.

The features of the P450 molecular species involved in drug metabolism are mentioned below. At first, CYP1A and CYP1 B are involved in the metabolic activation of heterocyclic amine contained in cancerogenic polycyclic aromatic hydrocarbon or singe, and is induced by polycyclic aromatic hydrocarbon. These enzymes are involved not only in oxidation of alcohol but also in metabolic activation of dimethylnitrosoamine and the like. P450 belonging to CYP3A metabolizes various drugs including 6- β -hydroxylation of testosterone. P450 belonging to CYP3A is present in human hepatic microsome in the largest amount and also expresses drug interaction when used together with other drugs that are also metabolized by CYP3A. Thus, P450 is also clinically attended. CYP3A7 exist specifically in human fetuses and CYP3A4 exist specifically in adults. The enzyme corresponding to CYP3A7 in human fetuses has not been identified in mouse or rat fetuses. The teratogenicity of thalidomide, which is detected in humans but is not detected in mice and rats, may be associated with the metabolic activation by CYP3A7 that is expressed in human fetuses, but this has not yet been revealed.

Wells et al. have found in a rabbit that the administration of acetylsalicylic acid which is a cyclooxygenase inhibitor suppresses the teratogenicity of thalidomide (The Journal of Pharmacology and Experimental Therapeutics, 277, 1649-1658, 1996), and have proved that cyclooxygenase generates an active reaction intermediate which would cause the teratogenicity. If a mouse or rat which excessively expressed cyclooxygenase were found, the thalidomide-induced sufferings might have been prevented.

In recent years, the analysis of gene functions in mammalian species makes progress by means of transgenic animals and gene targeting. Transgenic animals play important roles in the analysis of the gene function as well as in the analysis of the mechanism of diseases resulting from the interaction between the gene and other genes. A transgenic animal has a gene which was introduced into it or an embryonic line of its ancestor at the initial stage of development (usually, the single cell stage). Wagner et aL ("Proceedings of National Academy of Science, U.SA.," Vol.78, pp. 5016, 1981) and Stewart et al. ("Science," Vol.217, pp.1046, 1982) describe transgenic mice comprising a human globin gene. Constantini et al. (1981, "Nature," Vol.294, pp.92, 1981) and Lacy et al. ("Cell," Vol. 34, pp. 343, 1983) describe a transgenic mouse comprising a rabbit globin gene. McKnight et al. ("Cell," vol. 34, pp. 335, 1983) describe a transgenic mouse comprising a transferrin gene. Brinstar et al. (983, "Nature," Vol. 306, pp. 332, 1983) describe a transgenic mouse comprising a functionally introduced immunoglobulin gene.

The present inventors have heretofore produced a human P450 transgenic mouse (Yong Li et al, Archives of Biochemistry and Biophysics, Vol. 329, No. 2, 235-240, 1996). The CYP3A7 protein metabolically activates Aflatoxin B1. Because the resultant active metabolic product damages DNA, the ability of activation can be understood by application of this enzyme to the mutagenicity test using bacteria. When human P450 CYP3A transgenic mouse hepatic microsome was applied to the mutagenicity test using Salmonella TA100, this enzyme efficiently activated Aflatoxin B1 than the hepatic microsome of a wild-type mouse.

The ability for detecting teratogenicity in the human P450 CYP3A transgenic mouse is also interesting, but this detection of teratogenicity has not yet been successful. This could be caused by a low expression of the CYP3A7 protein in the human P450 CYP3A transgenic mouse fetus.

An object to be solved by the present invention is to provide a transgenic animal which is useful in a test for forecasting fetal toxicity and teratogenicity of drugs, and a recombinant gene for introduction which can be used in the production of said transgenic animal.

In order to attain the above object, the present inventors have conducted concentrated studies. At first, a transgene having SV40 poly(A) addition signal and a part (42 bp) of chicken beta globin gene insulator sequence was constructed by using CYP1A1, CYP1B1, CYP2E1, CYP3A7, COX-1, and COX-2 as human drug metabolizing enzyme genes and ligating them to the downstream of the CMV enhancer and the human EF1 α promoter. Subsequently, a transgenic mouse was produced in accordance with a conventional technique in which equimolar mole of these transgenes was mixed with each other, the resultant mixture was introduced into a fertilized mouse egg, and the gene-introduced fertilized egg was then transplanted into a mouse oviduct. The genotype of the thus produced transgenic mouse was confirmed. As a result, the present inventors have succeeded in obtaining a transgenic mouse into which 2 to 5 types of human drug metabolizing enzyme genes have been introduced. The present invention has been completed based on these findings.

Thus, the present invention provides a recombinant gene comprising: (1) a gene encoding human P450 or a mutant gene thereof; (2) a human EF1 α promoter, (3) a chicken β-globin insulator sequence or a part thereof; and (4) an SV40 poly(A) addition signal.

Preferably, the recombinant gene of the present invention is capable of expressing human P450 or a mutant protein thereof in a non-human animal cell.

According to another aspect, the present invention provides a recombinant gene comprising: (1) a gene encoding human cyclooxygenase (PGH synthase) or a mutant gene thereof; (2) a human EF1 α promoter; (3) a chicken β-globin insulator sequence or a part thereof; and (4) an SV40 poly(A) addition signal.

Preferably, the recombinant gene of the present invention is capable of expressing human cyclooxygenase or a mutant protein thereof in a non-human animal cell.

According to a further aspect, the present invention provides a recombinant vector which comprises the recombinant gene of the present invention.

According to a further aspect, the present invention provides a transformant which comprises the recombinant vector of the present invention.

According to a further aspect, the present invention provides a transgenic non-human animal, a progeny thereof, or a part thereof, into which several genes which are selected from the human P450 gene or a mutant gene thereof and/or the human cyclooxygenase gene or a mutant gene thereof, have been introduced.

According to preferred embodiments, the present invention provides:
the transgenic non-human animal, a progeny thereof, or a part thereof, wherein the introduced human P450 gene or a mutant gene thereof and/or the human cyclooxygenase gene or a mutant gene thereof is expressed in the body of the animal;
the transgenic non-human animal, a progeny thereof, or a part thereof, wherein the introduced human P450 gene or a mutant gene thereof and/or the human cyclooxygenase gene or a mutant gene thereof is expressed in the body of a fetus;
the transgenic non-human animal, a progeny thereof, or a part thereof, wherein the human P450 gene or a mutant gene thereof is introduced as a recombinant gene comprising: (1) a gene encoding human P450 or a mutant gene thereof; (2) a human EF1 α promoter, (3) a chicken β -globin insulator sequence or a part thereof; and (4) an SV40 poly(A) addition signal; and
the transgenic non-human animal, a progeny thereof, or a part thereof, wherein the human cyclooxygenase gene or a mutant gene thereof has been introduced as a recombinant gene comprising: (1) a gene encoding human cyclooxygenase (PGH synthase) or a mutant gene thereof; (2) a human EF1 α promoter; (3) a chicken β -globin insulator sequence or a part thereof; and (4) an SV40 poly(A) addition signal.

Preferred embodiments of the present invention provide:
the transgenic non-human animal, a progeny thereof, or a part thereof, wherein the human P450 gene is the CYP1A1 gene, CYP1B1 gene, CYP2E1 gene, or CYP3A7 gene; and
the transgenic non-human animal, a progeny thereof, or a part thereof, wherein the human cyclooxygenase gene is the cyclooxygenase-1 (COX-1) gene or cyclooxygenase-2 (COX-2) gene.

Preferred embodiments of the present invention provide the transgenic non-human animal, a progeny thereof, or a part thereof, which is selected from the group consisting of:
a transgenic non-human animal into which the COX-1 gene and the COX-2 gene have been introduced;
a transgenic non-human animal into which the CYP1A1 gene, CYP1B1 gene, CYP3A7 gene, and COX-2 genes have been introduced;
a transgenic non-human animal into which the CYP1B1 gene, COX-1 gene, and the COX-2 gene have been introduced; and
a transgenic non-human animal into which the CYP1A1 gene, CYP1B1 gene, CYP3A7 gene, COX-1 gene, and COX-2 gene have been introduced

In the present invention, a non-human animal is preferably selected from the group consisting of mouse, rat, hamster, guinea pig, rabbit, dog, cat, horse, cattle, sheep, pig, goat, monkey, chicken, quail, vinegar fly, and nematode, with mouse being particularly preferred.

According to a further aspect, the present invention provides a screening method for forecasting fetal toxicity and teratogenicity wherein the transgenic non-human animal, a progeny thereof, or a part thexeof according to the present invention is used.

Preferably, the screening method is provided wherein a test compound is administered to the transgenic non-human animal of the present invention, a progeny thereof, or a part thereof according to the present invention, and the expression of fetal toxicity and teratogenicity is observed

According to a further aspect, the present invention provides:
a substance which is judged to possess no fetal toxicity and no teratogenicity in the screening method of the present invention; and
a pharmaceutical comprising the substance which is judged to possess no fetal toxicity and no teratogenicity in the screening method of the present invention.

The pharmaceutical according to the present invention is preferably a preventive and therapeutic agent for the central nervous system disorders, psychiatric disorders, renal diseases, bone diseases, articular diseases, lung diseases, arteriosclerosis, heart diseases, diabetes, digestive system diseases, infectious diseases, allergic diseases, endocrine diseases, mental deterioration, or cancer.

### Brief Description of the Drawings

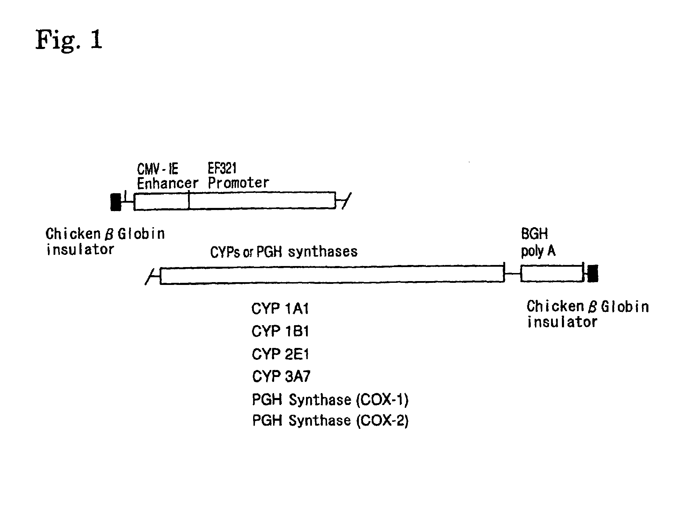

Fig. 1 shows a construction of an expression vector which comprises a gene for expressing the drug metabolizing enzyme produced in the Examples.
Fig. 2 shows constructions of expression vectors, pBSEFPINS42-3 and pBSEFPINS42-5, into which a gene for expressing the drug metabolizing enzyme is inserted
Fig. 3 shows a construction of DNA probe which comprises gene sequences for each of 6 types of drug metabolizing enzymes.
Fig. 4 shows the result of Southern hybridization which identifies the transgene in the transgenic mouse produced in the Examples. 110 generated mice were analyzed and, as a result, it was found that several genes were introduced into 4 mice. In Fig. 4, N represents a negative control, P represents a positive control, 3m represents COX-1 and COX-2 positive, 16m represents 1A1, 1B1, 3A7, and COX-2 positive, 34P represents 1B1, COX-1 and COX-2 positive, and 36P represents 1A1, 1B1, 3A7, COX-1, and COX-2 positive.
Fig. 5 shows the position of the PCR primer for detecting the genes of each of 6 types of drug metabolizing enzymes, and the chain length of the generated gene fragment
Fig. 6 shows the result of PCR for detecting the presence of the transgene in the production of the transgenic mouse F1 in the Examples. In Fig. 6, N represents a negative control, P represents a positive control, 16m represents 1A1, 1B1, 3A7, and COX-2 positive in individual 3 and individual 4, 34P represents 1B1, COX-1, and COX-2 positive in individual 1, and 36P represents 1A1, 1B1, 3A7, COX-1, and COX-2 positive in individual 1 and individual 4.

### Best Mode for Carrying out the Invention

The embodiments of the present invention are described in detail.

### (1) Recombinant gene of the invention

The present invention relates to a recombinant gene comprising: (1) a gene encoding human P450 or a mutant gene thereof; (2) a human EF1 α promoter, (3) a chicken β-globin insulator sequence or a part thereof; and (4) an SV40 poly(A) addition signal; and
a recombinant gene comprising (1) a gene encoding human cyclooxygenase (PGH synthase) or a mutant gene thereof; (2) a human EF1 α promoter; (3) a chicks β -globin insulator sequence or a part thereof; and (4) an SV40 poly(A) addition signal.

The recombinant gene of the present invention comprises a gene encoding human P450 (or a mutant gene thereof) or a gene encoding human cyclooxygenase (PGH synthase) (or a mutant gene thereof) downstream of a human EF1 α promoter sequence. Production of a transgenic mouse using a recombinant gene having such a construction as a transgene enables the production of a transgenic animal which efficiently expresses the transgene.

Among genes encoding human P450, the use of P450 genes which are involved in drug metabolism is preferable in the present invention. More specifically, a molecular species belonging to the CYP1-CYP3 families is preferred, and examples thereof include: CYP1A and CYP1B, which are involved in metabolic activation of heterocyclic amine contained in cancerogenic polycyclic aromatic hydrocarbon or singes, and induced by polycyclic aromatic hydrocarbon; CYP3A, which performs metabolization on various drugs including 6- β -hydroxylation of testosterone; and CYP2E, which is involved in oxidation of alcohol and metabolic activation of dimethylnitrosoamine.

Specific examples of the human 450 genes include, but are not limited to, the CYP1A1 gene, CYP1B1 gene, CYP2E1 gene, and CYP3A7 gene.

Examples of genes encoding the human cyclooxygenase (PGH synthase) include, but are not limited to, the cyclooxygenase-1 (COX-1) gene or cyclooxygenase-2 (COX-2) gene.

The gene encoding human P450 or the gene encoding human cyclooxygenase (PGH synthase) used in the present invention may be a mutant gene. Preferably, a mutant protein, which is encoded by such a mutant gene, maintains a function equivalent to or higher than that of the wild-type protein.

Specific examples of the mutant gene include a gene obtained by deleting a portion of the nucleotide sequence in the wild-type gene, a gene obtained by substituting a nucleotide sequence of the reporter gene with another nucleotide sequence, and a gene obtained by inserting another nucleotide sequence into the reporter gene. The number of nucleotides subjected to deletion, substitution or addition is not particularly limited, and it is generally about 1 to 60, preferably about 1 to 30, and more preferably about 1 to 15. This provides a protein wherein one to several (for example, 1 to 20, preferably 1 to 10, and more preferably 1 to 5) amino acids are substituted, deleted, added, and/or inserted into the amino acid sequence of the wild-type protein.

The mutant gene can be produced by any conventional method in the art, such as chemical synthesis, genetic engineering or mutagenesis. Specifically, a wild-type gene is brought into contact with a mutagenic agent, irradiated with ultraviolet, or subjected to genetic engineering techniques such as PCR Thus, a mutant gene can be obtained Site-directed mutagenesis, which is one of genetic engineering techniques, is particularly useful since it allows specific mutations to be introduced into specific sites, and it can be carried out in accordance with the method described in Molecular Cloning: A laboratory Manual (2^{nd} ED., Cold Spring Harbor Laboratory, Cold Spring Harbor, NY., 1989), Current Protocols in Molecular Biology (Supplement 1 to 38, John Wiley & Sons (1987-1997)), and the like.

The recombinant gene of the present invention comprises a human EF1 α promoter. The human EF1 α promoter is known and described in, for example, Kim D. W., Uetsuki T., Kaziro Y., Yamaguchi N., Sugano S., Use of the human elongation factor 1 alpha promoter as a versatile and efficient expression system, Gene, 1990, 16. In the recombinant gene of the present invention, the human EF1 α promoter is incorporated upstream of the gene encoding human P450 (or a mutant gene thereof) or the gene encoding human cyclooxygenase (PGH synthase)(or a mutant gene thereof), and these genes are positioned under the control of the human EF1 α promoter. The recombinant gene of the present invention may comprise an enhancer sequence upstream of the promoter sequence. Examples of enhancer sequences which can be used include, but are not limited to, the CMV enhancer.

The recombinant gene of the present invention comprises a chicken β-globin insulator sequence or a portion thereof. "Insulator sequence" refers to a gene sequence, in a transgenic animal, which prevents the inhibition of gene expression resulting from a "position effect" The insulator sequence is expected to be a barrier against the influence of cis-elements existing in the neighborhood.

The position of the insulator sequence or a part thereof in the recombinant gene of the present invention is not particularly limited. It may be positioned on the 5' side (upstream) or the 3' side (downstream) of the transgene (i.e., the gene encoding human P450 (or a mutant gene thereof) or the gene encoding human cyclooxygenase (PGH synthase) (or a mutant gene thereof)). The insulator sequence may be present in both thereof.

The recombinant gene of the present invention further comprises the SV40 poly(A) addition signal. Insertion of such a poly(A) addition signal sequence enables the termination of messenger RNA transcript of interest

Preferred embodiments of the recombinant gene of the present invention include a recombinant gene which comprises: from the 5' toward the 3', the chicken β-globin insulator sequence or a part thereof; the CMV enhancer, the human EF1 α promoter, the gene encoding human P450 (or a mutant gene thereof) or the gene encoding human cyclooxygenase (PGH synthase) (or a mutant gene thereof); the SV40 poly(A) addition signal sequence; and the chicken β -globin insulator sequence or a part thereof.

The recombinant gene of the present invention can be produced by any known gene recombinant techniques or any method in accordance therewith.

### (2) Recombinant vector of the invention and transformant having the recombinant vector

Further, the present invention relates to a recombinant vector containing the recombinant gene of the present invention and a transformant having the recombinant vector.

Specific examples of vectors when a bacterium is used as a host include, but are not limited to, pBTrP2, pBTac1 and pBTac2 (commercially available from Boehringer Mannheim), pKK233-2 (manufactured by Pharmacia), pSE280 (manufactured by Invitrogen), pGEMEX-1 (manufactured by Promega), pQE-8 (manufactured by QIAGEN), pQE-30 (manufactured by QIAGEN), pKYP10 (Japanese Patent Application Laying-Open No. 58-110600), pKYP200 [Agrc. Biol. Chem., 48, 669 (1984)], PLSA1 [Agrc. Blo1. Chem., 53,277(1989)], pGEL1 [Proc. Natl. Acad Sci. USA, 82, 4306 (1985)], pBluescrlptII SK+ and pBluescriptII SK(-) (manufactured by Stratagene), pTrS30 (FERMBP-5407), pTrS32 (FERM BP-5408), pGEX (manufactured by Pharmacia), pET-3 (manufactured by Novagen), pTerm2 (US4686191, US4939094, and US5160735), pSupex, pUB110, pTP5, pC194, and pUC18 [Gene, 33, 103 (1985)], pUC19 [Gene, 33, 103 (1985)], pSTV28 (manufactured by Takara Shuzo Co., Ltd.), pSTV29 (manufactured by Takara Shuzo Co., Ltd), pUC118 (manufactured by Takara Shuzo Co., Ltd.), pPA1 (Japanese Patent Application Laying-Open No. 63-233798), pEG400 [J. Bacteriol., 172, 2392 (1990)], and pQE-30 (manufactured by QIAGEN).

Specific examples of vectors when yeast is used as a host include, but are not limited to, YEp13 (ATCC37115), YEp24 (ATCC37051), Ycp5O (ATCC37419), pHS19, and pHS15.

Specific examples of vectors when an animal cell is used as a host include, but are not limited to, pcDNAI and pcDM8 (commercially available from Funakoshi), pAGE107 [Japanese Patent Application Laying-Open No. 3-22979; Cytotechnology, 3, 133 (1990)], pAS3-3 (Japanese Patent Application Laying-Open No. 2-227075), pCDM8 [Nature, 329, 840 (1987)], pcDNAI/AmP (manufactured by Invitrogen), pREP4 (manufactured by Invitrogen), pAGE103 [J-Blochem., 101, 1307 (1987)], and pAGE210.

In the production of a transformant, any cell can be used as a host as long as the gene of interest can be expressed. Usable examples thereof include: bacteria such as *Escherichia, Serratia, Coryncbacterium, Brevibacterium, Pseudomonas, Bacillus,* and *Mycobacterium;* yeast such as *Kluyveromyces, Saccharomyces, schizosaccharomyces, Trichosporon,* and *Schwanniomyces',* animal cells such as Namalwa cell, COS1 cell, COS7 cell, and CHO cell; plant cells; and insect cells such as Sf9 cell, Sf21 cell, and High5 cell.

A method for introducing a recombinant vector into a host can be suitably selected according to type of host and the like. Examples of methods for introducing recombinant vectors into bacterial cells include a method employing calcium ion, and the protoplast method. Examples of methods for introducing recombinant vectors into yeast include electroporation, spheroplast, and the Lithium Acetate method. Examples of methods for introducing recombinant vectors into animal cells include electroporation, the Calcium Phosphate method, and lipofection.

### (3) Transgenic non-human animal of the invention

The present invention further relates to a transgenic non-human animal, a progeny thereof, or a part thereof into which several genes selected from the human P450 gene or a mutant gene thereof and/or the human cyclooxygenase gene or a mutant gene thereof have been introduced. According to a preferred embodiment of the present invention, the introduced human P450 gene or a mutant gene thereof and/or the human cyclooxygenase gene or a mutant gene thereof is expressed in the body of the animal, and more preferably expressed in the body of the fetus.

In the transgenic animal of the present invention, a recombinant gene (hereinafter this may be referred to as a "transgene") having the human P450 gene or a mutant gene thereof and/or the human cyclooxygenase gene or a mutant gene thereof (hereinafter, this may be referred to as a "target gene") incorporated therein so as to be expressible in the body of the animal, has been introduced.

At first, the transgene used in the production of the transgenic animal of the present invention is described.

In the transgene used in the present invention, a target gene is present downstream of a promoter sequence which can work in non-human animals. Such a construction enables the expression of the target gene in non-human animal cells. Thus, in the transgene used in the present invention, the target gene is located so as to be under the control of the promoter described above.

The promoter sequence used in the transgene is not particularly limited as long as it can work in non-human animals.

Examples of promoters that are expressible in non-human animals usable herein include: gene promoters derived from viruses (e.g., cytomegalovirus, Moloney leukemia virus, JC virus, and breast cancer virus), and promoters derived from various mammalian species (e.g., human, rabbit, dog, cat, guinea pig, hamster, rat, and mouse). Promoters derived from various mammalian species usable herein include, for example, promoters of albumin, endothelin, osteocalcin, muscle creatine kinase, collagen type I and II, cyclic AMP-dependent protein kinase β subunit (The Journal of Biological Chemistry (vol. 271, No. 3, pp 1638-1644, 1996), atrial natriuretic factor, dopamine β - hydroxylase, neurofilament light chain (The Journal of Biological Chemistry, Vol. 270, No. 43, pp 25739-25745, 1995 and Vol.272, No.40, pp25112-25120, 1997)), metallothionein, tissue inhibitor of metalloproteinase-1, smooth muscle α-actin, polypeptide chain elongation factor 1 α (EF-1 α), β - actin, α -myosin heavy chain and β -myosin heavy chain, myosin light chain 1 and myosin light chain 2, myelin base protein, serum amyloid P component, and renin.

In addition to the above promoters, for example, promoters, which are described in the literature such as Molecular Medicine (occasional extra number), "Disease Model Mouse Manual," Ken-ichi Yamamura, Motoya Katsuki, and Shin-ichi Aizawa (ed.), NAKAYAMA SHOTEN CO., LTD. can be used.

Preferable promoters used in the present invention include human EF1 α promoter which is described in the Examples in the present specification, and further include the following.

### (1) β -Actin promoter

It is generally used as a combination of the CMV enhancer and the β -actin promoter. Examples include pCAGGS, chicken beta-actin promoter, cytomegalo virus enhancer, beta-actin intron, and bovine globin poly-adenylation signal. See H. Niwa, K. Yamanami, J. Miyazaki, Gene, 108, (1991) 193-199.

### (2) CMV promoter

It is generally used as a combination of the CMV enhancer and the CMV promoter. See Janet A. Sawicki et al., Experimental Cell Research 244,367-369 (1998).

### (3) Metallothionein promoter

See Establishment of Transgenic Mice Carrying Human Fetus-Specific CYP3A7," Yong Li, et al, Archives of Biochemistry and Biophysics, Vol. 329, No. 2, 235-240, 1996

### (4) Apolipoprotein E promoter

A promoter which is intended for the expression in fetal liver. See Simonet et aL, 1993, J. Biol. Chem., 268, 8221-8229.

The transgene of the present invention may contain an enhancer sequence upstream of the promoter sequence. Enhancer sequences usable herein include the above-mentioned CMV enhancer.

The transgene of the present invention may contain an insulator sequence or a part thereof. "Insulator sequence" refers to a gene sequence, in a transgenic animal, which prevents the inhibition of gene expression resulting from a "position effect." The insulator sequence is expected to be a barrier against the influence of cis-elements existing in the neighborhood.

The position of the insulator sequence or a part thereof is not particularly limited, and it can be located on the 5' side (upstream) and/or the 3' side (downstream) of the transgene (ie., a target gene). Preferably, the insulator sequence or a part thereof is located upstream of the promoter sequence on its 5' side (when the enhance sequence is present, it is located upstream thereof) and downstream of the poly(A) addition signal sequence on its 3' side which is described below.

In addition to the chicken beta-globin-derived insulator sequence as mentioned in the Examples of the present specification, insulator sequences which can be used in the transgene of the present invention include, but are not limited to, the following.
(1) scs and scs' sequences of vinegar flies (Rebecca Kellum and Paul Schedl, Cell, Vol. 64, 941-950, March 8, 1991)
(2) Insulator sequence of vinegar flies gypsy transposon (Holdrige, C., and D. Dorsett, 1991 Mol. Cell. Biol. 11: 1894-1900)
(3) Sea urchin arylsulfatase insulator sequence (Koji Akasaka, et al., Cellular and Molecular Biology 45 ( 5 ), 555-565,1999)
(4) Human T-cell receptor α/δ locus BEAD element (Zhong, X. P., and M. S. Krangel, 1997 Proc. NatuL Acad. Sci. USA)
(5) Human apolipo-protein B-100 (apoB) matrix attachment site (Namciu et al, 1998, Mol. Cell. Biol. 18: 2382-2391)

The transgene used in the present invention may contain a poly(A) addition signal sequence downstream of the target gene. Insertion of the poly(A) addition signal sequence enables the termination of messenger RNA transcript of interest.

An specific example of poly(A) addition signal sequence includes, but is not limited to, the SV40 poly(A) addition signal.

The transgene used in the present invention can be produced by any method known in the art or methods in accordance therewith.

Examples of the transgene preferably used in the present invention include:
a recombinant gene comprising: (1) a gene encoding human P450 or a mutant gene thereof; (2) a human EF1 α promote; (3) a chicken β -globin insulator sequence or a part thereof; and (4) an SV40 poly(A) addition signal; and
a recombinant gene comprising: (1) a gene encoding human cyclooxygenase (PGH synthase) or a mutant gene thereof; (2) a human EF1 α promoter; (3) a chicken β -globin insulator sequence or a part thereof; and (4) an SV40 poly(A) addition signal Details of these recombinant genes are as described above in the present specification.

Preferred examples of the gene encoding human P450 or the gene encoding human cyclooxygenase (PGH synthase) are as described above in the present specification.

The transgenic animal of the present invention includes an embryo generated from a fertilized egg of a non-human animal, into which the transgene has been introduced, and a fetus generated by transplanting the embryo into a womb or oviduct of a corresponding non-human animal. These animals are transgenic non-human animals which express a target gene.

The present invention also relates to a part of the transgenic non-human animal Examples of the part of non-human animal include intracellular organelles, cells, tissues, organs, heads, fingers, hands, legs and feet, abdomens, and tails.

Examples of non-human animals used herein include, but are not limited to: non-human mammalian species such as rabbit, dog, cat, guinea pig, hamster, mouse, rat, sheep, goat, pig, horse, cattle, and monkey; birds such as chicken and quail; insects such as vinegar fly; and nematode. Preferably, non-human animals are non-human mammalian species, and rodent mammalian species (Rodentia) such as mouse, rat, and guinea pig are more preferred, with mouse and rat being particularly preferred. Examples of mouse include the lineages of the pure-line C57BL/6, DBA/2, and BALB/c, the lineages of the hybrid-line B6C3F1 and B6D2F1, and the lineage of the closed colony ICR Specific examples of rat include Wister and SD rats.

In one preferred embodiment of the present invention, the transgenic non-human animals of the present invention may have DNA incorporated therein so as to allow the target gene to be expressed in a specific site.

The phrase "to be expressed in a specific site" used herein refers to the fact that the target gene can be expressed in a specific location in intracellular sites, intracellular organelles, cells, tissues, or organs.

Intracellular specific sites include the axis cylinder of neurocytes and the like. Intracellular organelles include, for example, nuclei, mitochondria, Golgi apparatuses, endocytoplasmic reticulums, ribosomes, and cell membranes. Examples of cells include: hepatocytes, splenocytes, neurocytes, gliocytes, pancreatic β-cells, myeloma cells, mesangium cells, Langerhans's cells, epidermal cells, epithelial cells, endothelial cells, fibroblasts, fibrocytes, myocytes, adipocytes, immunocytes, megakaryocytes, synoviocytes, chondrocytes, osteocytes, osteoblasts, osteoclasts, alveolar epithelial cells, hepatocytes, or interstitial cells of mammalian species; and their precursor cells, stem cells, or cancer cells. Tissues include any tissue in which the above-mentioned cells are present, for example, brain (e.g., amygdaloid nucleus, basal ganglia, hippocampus, hypothalamus, cerebral cortex, medulla, cerebellum, and epiphysis), spinal cord, hypophysis, stomach, sexual gland, thyroid gland, gallbladder, bone marrow, adrenal gland, skin, muscle, lung, large intestine, small intestine, duodenum, rectum, blood vessel, thymus gland, submandibular gland, peripheral blood, prostate gland, spermary, ovary, placenta, uterus, bone, articulation, and skeletal muscle. Alternatively, it may be a hemocyte or a cultured cell of the above-mentioned cells. Organs include heart, kidney, pancreas, liver, and spleen.

According to one preferred embodiment, the transgenic non-human animals of the present invention may have DNA incorporated therein so as to allow the target gene to be expressed at specific stages.

The term "specific stages" used herein refers to specific stages of embryo generation, birth, or specific phases from the embryo generation until death, and the like. Accordingly, a specific stage may be any stage during embryo generation including a stage of the introduction of the heterogenous gene, on the basis of hours, days, weeks, months, and years being passed after birth.

In order to express the target gene at a specific stage in the transgenic non-human animal according to the present invention, an expression vector, which contains a promoter region capable of expressing a protein at specific stages, and a signal sequence capable of expressing a protein at specific stages, or the like is used to produce a transgenic non-human animal having a target gene.

The target gene can be expressed at specific stages by constructing a system for the induction of protein expression or by administering a protein expression inducer to a non-human animal at specific stages. Examples of the protein expression-inducing system usable herein include an expression induction system which utilizes tetracyclin or ecdysone. The agent which is administered in that case is tetracycline or an analogue thereof or ecdysone or an analogue thereof. Also, the cre-loxP system utilizing a recombinase may be employed

Further, the target gene can be expressed at specific stages by incorporating genes or the like which are resistant to antibiotics such as tetracyclin, kanamycin, hygromycin and puromycin into an expression vector. The location of these resistant genes in the expression vector according to the present invention is not particularly limited. In general, it is preferably located downstream of the target gene or upstream of the promoter region or signal sequence.

The transgenic non-human animal according to the present invention can be produced by introducing the transgene into a subject animal. More specifically, by introducing the transgene into a fertilized egg, embryonic stem cell, somatic cell, sperm, or unfertilized egg of a subject non-human animal, a transgenic non-human animal having the gene incorporated into all the cell chromosomes including germinal cells can be obtained Introduction of transgenes into a fertilized egg, embryonic stem cell, somatic cell, sperm, or unfertilized egg must be carried out in such a way that the gene is present in all cell chromosomes including germinal and somatic cells of the subject non-human animal.

Offsprings of the transgenic non-human mammals wherein the transgene is integrated in the chromosomes of all the cells including germinal similarly possess the gene of interest. Homozygote animals having the transgene in both of the homologous chromosomes are obtained, and female and male rhereof were mated in such a manner that all offspring stably sustain the gene, thus confirming the possession of the genes. Thereby, the offspring can be reproduced and subcultured in a general breeding environment.

A method for producing the transgenic non-human mammals of the present invention is described below in more detail.

The transgenic non-human mammals of the present invention can be produced by introducing the transgene into, for example, a fertilized egg, and sperm and a germ cell containing a progenitor cell thereof, preferably at the early stage of embryo formation (more preferably at the single cell stage or amphicytula and before the 8-cell stage in general) in the generation of non-human mammals.

The fertilized egg to be used when introducing the transgene into the fertilized egg of the non-human mammals of interest or its progenitor is obtained by mating a male non-human mammals with a conspecific female. The fertilized egg can be obtained by natural mating, however, it is preferably obtained by artificially controlling the estrous cycle of the female non-human mammals and then mating it with a counterpart male. A preferred method for artificially controlling the estrous cycle of the female non-human mammals is carried out, for example, by first administering follicle stimulating hormone (pregnant mare serum gonadotropin) and then administering luteinizing hormone (human chorionic gonadotropin) by abdominal injection or the like. Preferably, the dose and the administration interval of hormone can be suitably determined based on the type of non-human mammals.

Examples of methods for introducing a transgene include conventional methods such as calcium phosphate method, electropulse method, lipofection method, coagulation method, microinjection method, particle gun method, and DEAE-dextran method. The transgene of interest is introduced into a somatic cell in accordance with the above mentioned introduction method, and this cell (or a nucleus thereof) is fused with the above germ cell in accordance with any known cell fusion technique. Thus, the transgenic non-human mammals of the present invention can be produced. The introduction of a transgene at the stage of amphicytula is carried out in such a way that the transgene is present in all germ and somatic cells of the subject animal.

After the transgene is introduced into the fertilized egg, the egg is artificially transplanted and implanted into a female non-human mammals. Thus, the transgenic non-human mammals having the transgene can be obtained. It is preferable that luteinizing hormone releasing hormone (LHRH) or an analogue thereof is administered, and then the female non-human mammals is mated with a male mammals, and thereby the transgene-introduced fertilized egg is artificially transplanted and implanted into a pseudopregnant female non-human mammalian species. The dose of LHRH or an analogue thereof and the stage of mating with a conspecific male after the administration can be suitably selected depending on the type of non-human mammals and the like.

Whether or not the transgene is successfully incorporated into the genomic DNA can be confirmed by extracting DNA from the tail of the offspring and examining by the Southern hybridization, PCR or the like.

If the transgene is present in the germ cell of the produced animal after the introduction of transgene, it indicates that the progeny of the produced animal maintains the transgene in all the germ cells and somatic cells. The offspring animal of this species, which inherited the transgene, has the transgene in all the germ cells and somatic cells.

A homozygote animal having the transgene in both homologous chromosomes is obtained, and the female is mated with the male, thereby reproducing and subculturing in such a manner that all the offspring have the transgene. The thus obtained offspring is also included in the animal according to the present invention.

Regarding the detailed production process of the transgenic animals, reference can be made to, for example, Manipulating the Mouse Embryo (Brigid Hogan et al, Cold Spring Harbor Laboratory Press, 1986), Gene Targeting, A Practical Approach (IRL Press at Oxford University Press (1993)), Bio Manual Series 8, Gene Targeting (Production of Mutant Mouse using ES Cell, YODOSHA CO., LTD. (1995)), and Experimental Manual for Development Engineering, A Method for Producing Transgenic Mouse (Kodansha Ltd. (1987)).

### (4) Use of the transgenic non-human animal of the invention

The present invention further relates to a screening method for forecasting fetal toxicity and teratogenicity wherein the transgenic non-human animal, a progeny thereof, or a part thereof as mentioned above is used. An embodiment of the screening method of the present invention includes a step of administering a test compound to the transgenic non-human animal of the present invention, a progeny thereof, or a part thereof, and observing the expression of fetal toxicity and teratogenicity.

The transgenic non-human animal of the present invention possesses several types of human drug metabolizing enzyme genes and is thus a useful model animal for evaluating fetal toxicity and teratogenicity of a test compound. Therefore, the transgenic non-human animal of the present invention or a part thereof can be used for administering a test compound to forecast fetal toxicity and teratogenicity of the test compound.

An embodiment of the method for screening a test compound using the transgenic non-human animal of the present invention is described below. The type of test compound to be used in the present invention is not particularly limited, and screening can be carried out on compounds having preventive and therapeutic effects on any diseases or disorders such as central nervous system disorders, psychiatric disorders, renal diseases, bone diseases, articular diseases, lung diseases, arteriosclerosis, heart diseases, diabetes, digestive system diseases, infectious diseases, allergic diseases, endocrine diseases, mental deterioration, or cancers.

Examples of test compounds include peptides, proteins, non-peptidic compounds, synthetic compounds, fermentative products, cell extracts, plant extracts, animal tissue extracts, and blood plasmas. These compounds may be novel or known compounds.

More specifically, the present invention provides a method for screening a target substance, wherein a test compound is administered to the transgenic animal of the present invention, a progeny thereof, or a part thereof; the transgenic animal of the present invention is compared with a control transgenic animal or a part thereof which is given no drug; and changes in the expression of fetal toxicity and teratogenicity in the transgenic animal or a part thereof is employed as an index.

The expression of fetal toxicity and teratogenicity can be observed by any known method or any method in accordance therewith.

Methods for administering a test compound to the transgenic non-human animal of the present invention usable herein include, for example, oral administration and intravenous injection. The dose of the test compound can be suitably selected depending on the administration method, the properties of the test compound, and the like.

The present invention further relates to a substance which possesses no fetal toxicity and no teratogenicity in the above-mentioned screening method.

The substance obtained by the screening method of the present invention is a compound which is selected from the test compounds described above and is forecasted to show no fetal toxicity and no teratogenicity. Accordingly, it can be put to pharmaceutical use as a safe and low toxic therapeutic and preventive agent Specifically, the present invention also relates to a pharmaceutical comprising a substance which possesses no fetal toxicity and no teratogenicity in the above-mentioned screening method.

The application of the pharmaceutical of the present invention is not particularly limited, and examples thereof include preventive and therapeutic agents for central nervous system disorders, psychiatric disorders, renal diseases, bone diseases, articular diseases, lung diseases, arteriosclerosis, heart diseases, diabetes, digestive system diseases, infectious diseases, allergic diseases, endocrine diseases, mental deterioration, or cancers.

The substance which was obtained by the screening method of the present invention, may form a salt. Salts of the substance include salts with physiologically acceptable acid (e.g., inorganic or organic acid) or base (e.g., alkali metal). A physiologically acceptable acid added salt is particularly preferred. Examples of such salts include: a salt with inorganic acid such as hydrochloric acid, phosphoric acid, hydrobromic acid, or sulfuric acid; and a salt with organic acid such as acetic acid, formic acid, propionic acid, fumaric acid, maleic acid, succinic acid, tartaric acid, citric acid, malic acid, oxalic acid, benzoic acid, methane sulfonic acid, and benzene sulfonic acid.

The substance obtained by the screening method can be orally administered in the form of, for example, an optionally sugar-coated tablet, capsule, elixir, or microcapsule. Alternatively, it can be parenterally administered in the form of an aseptic solution of the substance and water or other pharmaceutically acceptable liquid or an injection such as suspension. For production as a pharmaceutical, the substance can be mixed, for example, with a physiologically acceptable carrier, flavoring agent, excipient, vehicle, preservative, stabilizer, binder or the like in the unit dose form required in commonly accepted pharmaceutical preparations.

The amount of active ingredients is determined to bring a preparation to suitable volume in the specified range. Examples of usable additives, which can be mixed in the tablet, capsule, and the like include: binders such as gelatin, corn starch, Tragacanth, and gum Arabic; excipients such as crystalline cellulose; swelling agents such as corn starch, gelatin, and alginic acid; lubricants such as magnesium stearate; sweetening agents such as sucrose, lactose, or saccharin; and flavoring agents such as peppermint, oil of *Gaultheria adenothrix,* or cherry. When the formulated unit form is a capsule, a liquid carrier such as fat and oil can be further added to the material described above. An aseptic composition for injection can be formulated in accordance with commonly used pharmaceutical preparation by, for example, dissolving or suspending an active substance in a vehicle such as water, or naturally-occurring vegetable oils such as sesame oil- and palm oil Aqueous solutions for injection usable herein include, for example, isotonic solutions containing physiological saline, glucose, and other adjuvant (e.g., D-sorbitol, D-mannitol, and sodium chloride), and it may be used in combination with suitable solubilizers such as alcohol (e.g., ethanol), polyalcohol (e.g., propylene glycol and polyethylene glycol), or nonionic surfactant (e.g., Polysorbate 80™, HCO-50). Oily fluids usable herein include, for example, sesame oil and soybean oil, and may be used in combination with a solubilizer, such as benzyl benzoate or benzyl alcohol.

The therapeutic and preventive agents may contain, for example, a buffer (e.g., phosphate buffer and sodium acetate buffer), a soothing agent (e.g., benzalkonium chloride and procaine hydrochloride), a stabilizer (e.g., human serum albumin and polyethylene glycol), a preservative (e.g., benzyl alcohol and phenol), and an antioxidant. The prepared pharmaceutical composition such as a parenteral solution is generally filled into a suitable ampule.

The preparation thus obtained is safe and low in toxicity and, thus, can be administered to, for example, humans and other mammalian species (e.g., rat, rabbit, sheep, pig, cattle, cat, dog, and monkey). The dosage of the substance varies depending on the disease, the subject for administration, the route of administration, or the like. The compound is administered in amounts of generally about 0.001 to 100 mg, preferably about 0.01 to 50 mg, more preferably about 0.1 to 20 mg to an adult per day.

All of the contents disclosed in the specification of Japanese Patent Application No. 2001-47735, based on which the present application claims priority, is incorporated herein by reference as a part of the disclosure of the present application.

The present invention wi be described in more detail with reference to the following examples, but the present invention is not limited to these examples.

### Examples

### Example 1: Construction of expression vector for drug metabolizing enzyme gene fragments

At first, expression vectors for inserting cDNA of the drug metabolizing enzyme were constructed.

The *xba*I site of pBlueScript II sk (+) was cleaved and then blunt-ended using T4 DNA Polymerase. The *Xba*I-*Xho*I fragment of pIRESneo2 (Clontech) comprising the poly(A) addition signal was blunt-ended and then inserted. The resultant plasmid was cleaved with *Eco*RI and *Hind*III, and the CMV enhancer and the EF1 α promoter were similarly cleaved out with *Eco*RI and *Hind*III from pCE-EGFP-1 (Takada, T. et al., Selective production of transgenic mice using green fluorescent protein as a marker. Nature Biotech. 15: 458-461, 1997, distributed by Dr. Sugano, Division of Research in Cancer Virus, The Institute of Medical Science, The University of Tokyo), followed by insertion. Subsequently, the resultant plasmid was cleaved with *Not*I and treated with CIAP. Thereafter, 42 bp insulator sequence of the chicken β -globin gene (Adam C. Bell et. al., The Protein CTCF Is Required for the Enhancer Blocking Activity of Vertebrate Insulators, Cell, Vol. 98, 387-396, 1999) was synthesized and inserted. The direction of the insulator sequence was confirmed by reading the nucleotide sequence, the plasmid was cleaved with *Sal*I, and the synthetic insulator sequence was similarly inserted. "insulator sequence" refers to a gene sequence, in a transgenic animal, which prevents the inhibition of gene expression resulting from a "position effect". The insulator sequence is expected to be a barrier against the influence of cis-elements existing in the neighborhood. In this example, a vector comprising the same 42 bp insulator sequences on each of the 5' side and the 3' side of the gene fragment for expressing a drug metabolizing enzyme was constructed.

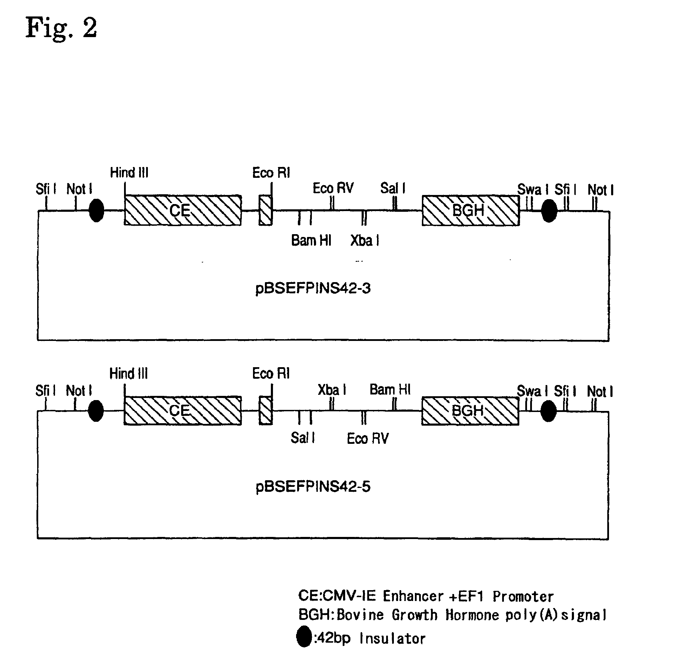

Thereafter, a multicloning site linker having a restriction site capable of incorporating each cDNA was synthesized and incorporated between the EF1 α promoter and the poly(A) addition signal. Fig. 2 shows a construction of expression vectors, pBSEFPINS42-3 and pBSEFPINS42-5, into which genes for expressing the drug metabolizing enzyme are inserted In both vectors, the multicloning site linker is inserted in the opposite direction. Insertion of the cDNAs of 6 different types of drug metabolizing enzymes individually into each vector enables the production of drug metabolizing enzyme gene fragment which can be driven by the EF1 α promoter.

### Example 2: Preparation of expression vector comprising drug metabolizing enzyme gene fragments

The cDNAs of CYP1A1, CYP1B1, CYP2E1, CYP3A7, PGHS-1(COX-1) and PGHS-2(COX-2), used as transgenes in this examples, are all known. The origin of these genes and the method for obtaining them are summarized in Table 1 below.

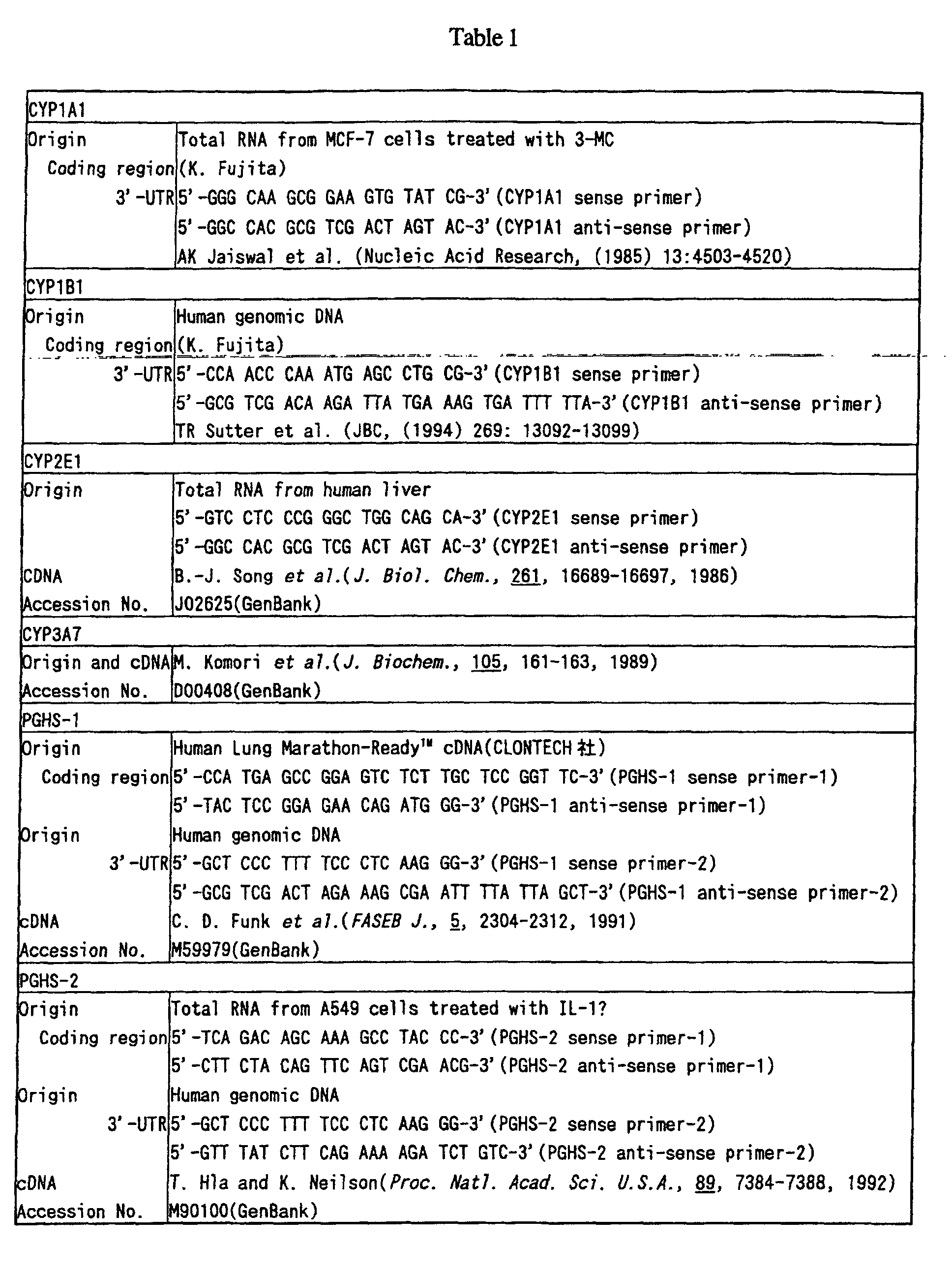

A plasmid containing CYP1A1 cDNA was cleaved with *Xba*I and *Xho*I*,* and subjected to agarose gel electrophoresis for separation. Thereafter, the cDNA portion was cleaved out and recovered. A pBS EFP INS42-3 was cleaved with *Xba*I and *Sal*I, and a CIAP-treated vector were prepared separately, and ligated to each other using the Ligation Kit Ver. 2 to obtain pBS EFP 1A1.

A plasmid containing CYP1B1 cDNA was cleaved with *Bam*HI and *Sal*I, and subjected to agarose gel electrophoresis for separation. Thereafter, the cDNA portion was cleaved out and recovered. A pBS EFP INS42-3 was cleaved with *Bam*HI and SalI*,* and a CIAP-treated vector was prepared separately, and ligated to each other using the Ligation Kit Ver. 2 to obtain pBS EFP 1B1.

A plasmid containing COX-1 cDNA was cleaved with *Hind*III and then blunt-ended using T4 DNA Polymerase. Then, the plasmid was cleaved with *Sal*I, and subjected to agarose gel electrophoresis for separation. Thereafter, the cDNA portion was cleaved out and recovered A pBS EFP INS42-3 fragment was cleaved with *Eco*RV and *Sal*I, and a CIAP-treated vector was prepared separately, and ligated to each other using the Ligation Kit Ver. 2 to obtain pBS EFP COX-1.

A plasmid containing CYP2E1 cDNA was cleaved with *Xho*I, and subjected to agarose gel electrophoresis for separation. Thereafter, the cDNA portion was cleaved out and recovered A pBS EFP INS42-5 fragment was cleaved with *Sal*I, and a CIAP-treated vector was prepared separately, and ligated to each other using the Ligation Kit Ver. 2 to obtain pBS EFP 2E1.

A plasmid containing CYP3A7 cDNA was cleaved with *Xho*I and *Bam*HI, and subjected to agarose gel electrophoresis for separation. Thereafter, the cDNA portion was cleaved out and recovered. A pBS EFP INS42-5 was cleaved with *Sal*I and *Bam*HI, and a CIAP-treated vector was prepared separately, and ligated to each other using the Ligation Kit Ver. 2 to obtain pBS EFP 3A7.

A plasmid containing COX-2 cDNA was cleaved with *Eco*RI and *Xho*l, and subjected to agarose gel electrophoresis for separation. Thereafter, the cDNA portion was cleaved out and recovered. A pBS EFP INS42-5 fragment was cleaved with *Eco*RI and *Sal*I, and a CIAP-treated vector was prepared separately, and ligated to each other using the Ligation Kit Ver. 2 to obtain pBS EFP COX-2.

### Example 3: Preparation of drug metabolizing enzyme gene fragments

pBS EFP 1A1 plasmid DNA was prepared in a large scale by the Alkali lysis method. This plasmid was cleaved with *Not*I, and about 4.5 kbp DNA fragment was separated by agarose gel electrophoresis and recovered by electroelution. Further, the product was purified by phenol/chloroform extraction, chloroform extraction, and ethanol precipitation. The purified product was centrifugated and then diluted with PBS(-) to obtain CYP1A1 gene fragment

pBS EFP 1B1 plasmid DNA was prepared in a large scale by the Alkali lysis method. This plasmid was cleaved with *Sfi*I, and about 6.8 kbp DNA fragment was separated by agarose gel electrophoresis and recovered by electroelution. Further, the product was purified by phenol/chloroform extraction, chloroform extraction, and ethanol precipitation. The purified product was centrifugated and then diluted with PBS(-) to obtain CYP1B1 gene fragment

pBS EFP COX-1 plasmid DNA was prepared in a large scale by the Alkali lysis method. This plasmid was cleaved with *Not*I, and about 4.6 kbp DNA fragment was separated by agarose gel electrophoresis and recovered by electroelution. Further, the product was purified by phenol/chloroform extraction, chloroform extraction, and ethanol precipitation. The purified product was centrifugated and then diluted with PBS(-) to obtain COX-1 gene fragment

pBS EFP CYP2E1 plasmid DNA was prepared in a large scale by the Alkali lysis method. This plasmid was cleaved with *Not*I, and about 3.7 kbp DNA fragment was separated by agarose gel electrophoresis and recovered by electroelution. Further, the product was purified by phenol/chloroform extraction, chloroform extraction, and ethanol precipitation. The purified product was centrifugated and then diluted with PBS(-) to obtain CYP2E1 gene fragment

pBS EFP CYP3A7 plasmid DNA was prepared in a large scale by the Alkali lysis method. This plasmid was cleaved with *Not*I, and about 4.0 kbp DNA fragment was separated by agarose gel electrophoresis and recovered by electroelution. Further, the product was purified by phenol/chloroform extraction, chloroform extraction, and ethanol precipitation. The purified product was centrifugated and then diluted with PBS(-) to obtain CYP3A7 gene fragment

pBS EFP COX-2 plasmid DNA was prepared in a large scale by the Alkali lysis method. This plasmid was cleaved with *Not*I, and about 6.4 kbp DNA fragment was separated by agarose gel electrophoresis and recovered by electroelution. Further, the product was purified by phenol/chloroform extraction, chloroform extraction, and ethanol precipitation. The purified product was centrifugated and then diluted with PBS(-) to obtain COX-2 gene fragment

The preparation method for the above 6 types of gene fragments are shown in Table 2. Six types of gene fragments were mixed at an equal mole to prepare a 3.9 ng/µl DNA PBS(-) solution (final concentration).

**Table 2**

| | cDNA | Cleaved-out fragment | Vector to be inserted | Site to be inserted | Size | Cleaving method | Injection fragment size |
|---|---|---|---|---|---|---|---|
| CYP1A1 | 2.5 kb | *Xba*I/*Xho*I | pBS EFP INS42-3 | *Xba*I/*Sal*I | 7.5 kb | *Sfi*I or *Not*I | 4.5 kb |
| CYP1B1 | 4.8 kb | *Bam*HI/*Sal*I. | | *Bam*HI/*Sal*I | 9.8kb | *Sfi*I | 6.8kb |
| COX-1 | 2.6kb | (*Hind*III)/*Xho*I | | *Eco*RV/*Sal*I | 7.6kb | *Sfi*I or *Not*I | 4.6kb |
| CYP2E1 | 1.7kb | *Xho*I/*Xho*I | pBS EFP INS42-5 | *Sal*I/*Sal*I | 6.7kb | *Sfi*I or *Not*I | 3.7kb |
| CYP3A7 | 2.0kb | *Xho*I/*Bam*HI | | *Sal*I/*Bam*HI | 7.0kb | *Not*I | 4.0kb |
| COX-2 | 4.4kb | *EcoR*I/*Xho*I | | *Eco*RI/*Sal*I | 9.4kb | *Sfi*I *or Not*I | 6.4 kb |
| Preparation method for 6 types of drug metabolizing enzyme gene fragments | | | | | | | |

### Example 4: Production of fertilized egg

The fertilized egg was produced by *in-vitro* fertilization in accordance with the technique by Toyoda et al. (Research on *in vitro* fertilization of mouse eggs, The Japanese Journal of Animal Reproduction, 16: 147-151, 1971). Specifically, PMSG and hCG (5 units) were administered to a female C57BL/6 mouse through intraperitoneal injection at 48 hour intervals. Thereafter, eggs were collected 16 to 18 hours later, and was inseminated (about 100 to 150 sperms/µl) with the C57BL/6 sperm (sperm was collected about 1.5 hour before egg collection). About 6 hours after insemination, discharge of the secondary polar body of egg and the presence of both pronucleus of male and female were confirmed, and only the fertilized eggs were selected. The obtained fertilized eggs at the pronucleus stage were cryopreserved by simple vitrification in accordance with the technique by Nakao et al. (Nakao K., Nakagata N., Katsuki M., Simple and efficient vitrification procedure for cryopreservation of mouse embryos, Exp. Anim., 1997, 46, 231-234). The frozen fertilized eggs were melted before the experiment and subjected to microinjection.

### Example 5: Microinjection of DNA

Microinjection of DNA into the pronucleus of the fertilized egg was carried out in accordance with the technique by Katsuki et al. (Experimental Manual for Development Engineering, A Method for Producing Transgenic Mouse (Kodansha Scientific, 1987)). The fertilized eggs were transferred to drops of modified Whitten's medium (mWM medium). Regarding injection into the nucleus, after the male pronucleus was confirmed under the phase-contrast microscope (Invert Scope D: Ziess), about 2 p1 of the purified DNA solution (prepared in Example 3) was injected. Regarding injection into cytoplasm, about 2 p1 of the DNA solution was injected into the cytoplasm. The surviving embryos were transferred into the mWM medium, and further subjected to culturing under 5% CO₂, 95% Air, and 37°C. Thereafter, the culture products were transplanted into the oviducts of pseudopregnant female mice of the ICR lineage, and implanted

### Example 6: Genotyping

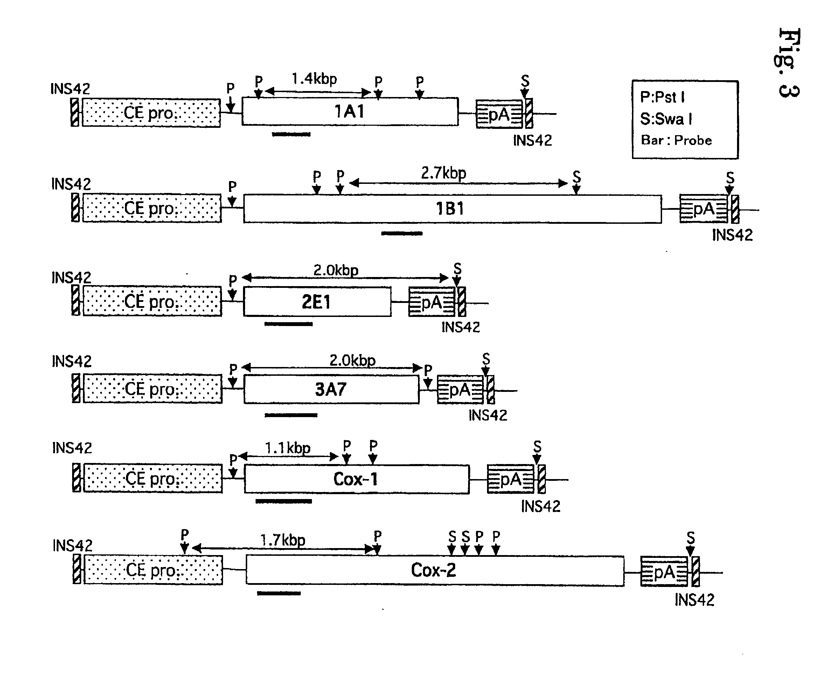

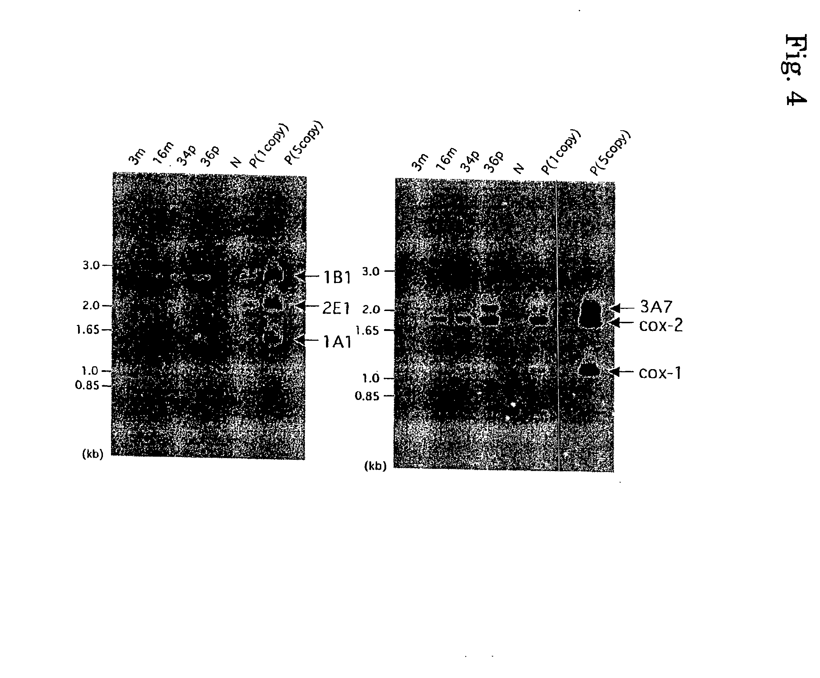

About 1 cm of the tail of a 4 week-old mouse was cleaved, placed in a solubilizing buffer containing pronase K and proteinase E, and was solubilized at 55°C overnight. DNA was obtained from the solubilized solution using a DNA extracting apparatus (manufactured by Toyobo Co., Ltd.). The obtained DNA was analyzed by Southern hybridization using a DNA probe containing gene sequences for the 6 types of drug metabolizing enzymes (labeled by a known radioisotope labeling technique)(Fig.3). A commercially available labeling kit (manufactured by Stratagene) was used for labeling. Approximately 10 µ g of DNA was completely cleaved with restriction enzymes *Pst*I and *Swa*I, subjected to electrophoresis in 0.8% agarose, and transferred to a nylon filter in accordance with the method described by Southern (1975, The Journal of Molecular Biology, Vol. 98, p.503). This filter was hybridized with a probe for 1 hour in the Quick Hybridization Buffer (manufactured by Toyobo Co., Ltd.), followed by washing twice at 2xSSC, 0.1% SDS at 65°C for 5 minutes each time and then twice for 30 minutes each time. As a result of the Southern hybridization, drug metabolizing enzyme genes were detected in 4 individuals among 110 offspring mice which were examined (Fig. 4).

The genes introduced into 4 transgenic mice are shown in Table 3 below.

**Table 3**

| Individual No. (sex) | Type of transgene |
|---|---|
| 3m (female) | COX-1, COX-2 |
| 16m (female) | 1A1, 1B1, 3A7, COX-2 |
| 34p (male) | 1B1, COX-1, COX-2 |
| 36p (male) | 1A1, 1B1, 3A7, COX-1, COX-2 |

Further, the above transgenic mice were mated with a mouse of C57BL/6 lineage to obtain their offsprings. The resultant offsprings were subjected to the same method as described above to confirm the presence of the transgenes. As a result, offsprings having the introduced transgene were obtained

### Example 7: Analysis of teratogenicity and fetal toxicity effects of thalidomide on transgenic (Tg) mouse

Frozen eggs of the transgenic mouse were prepared in accordance with "Example 4: Production of fertilized egg," and fertilized eggs of the transgenic mouse were cryopreserved. The frozen fertilized eggs were melted, and 20 eggs were respectively transplanted into the oviducts of pseudopregnant female mice of the ICR lineage, and implanted. On the 9th day of gestation, thalidomide was administered into the maternal peritoneal cavity. The effect of thalidomide was *tested at the dosage of: 100* mg/kg, *and 200* mg/kg. *A mixed solution of Tween 20 and* physiological saline (1:3) was used as a vehicle. On the day before the delivery, which is the 19th day of gestation, the maternal body was subjected to laparotomy to observe the conditions inside the womb and the presence of teratogenicity and fetal toxicity in the generated and grown fetus. Also, tissues were collected from the fetuses generated and grown in the womb, or vestige of fetal matter in the womb, DNA was extracted therefrom, and the genotype was assayed by PCR The results are summarized in Table 4.

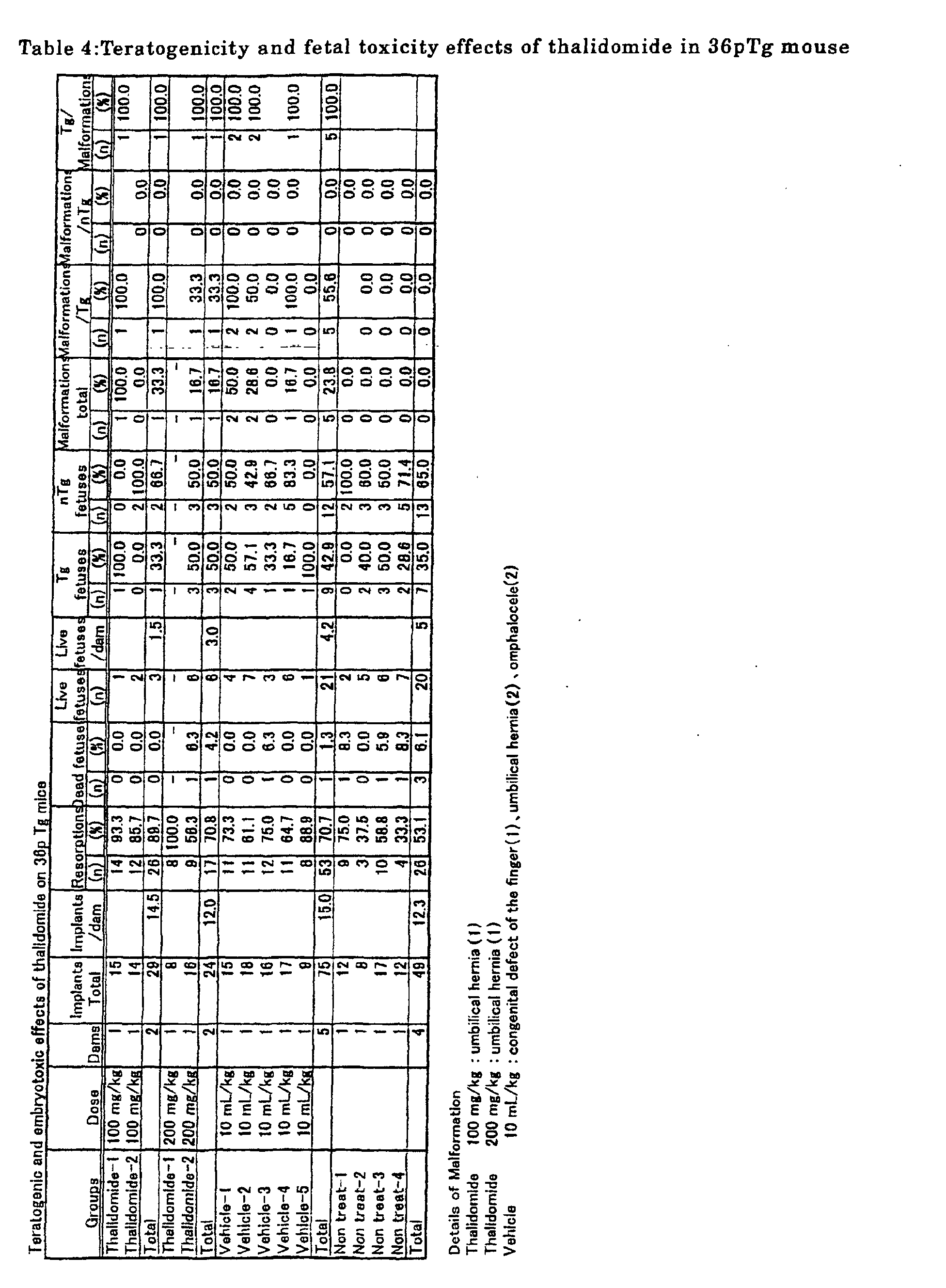

The results in Table 4 suggest the effects of the transgenes on the teratogenicity and fetal toxicity.

### Example 8: Typing by PCR of mouse (P450-6 mix mouse) F1 into which 6 types of drug metabolizing enzymes were introduced

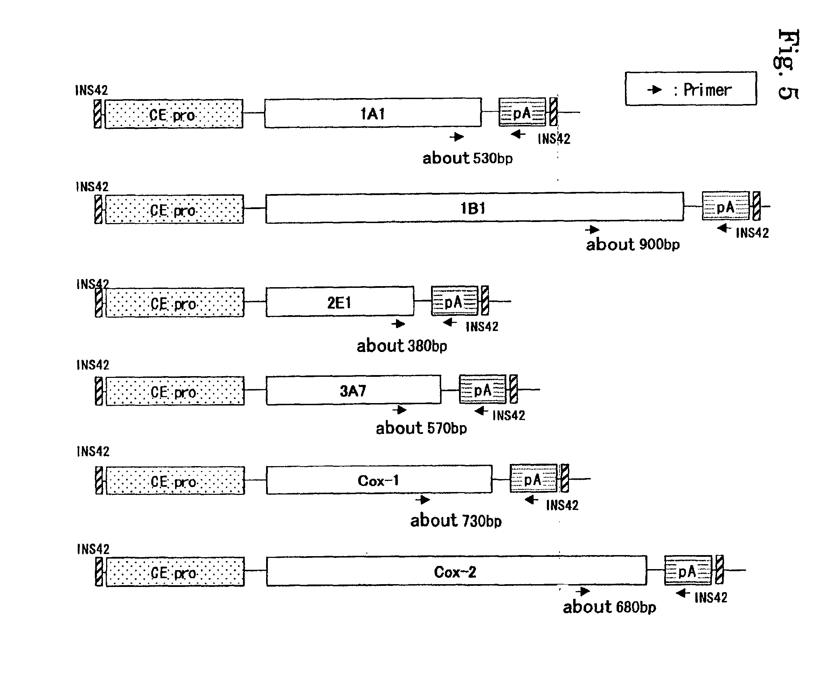

Genotyping of the offspring such as the obtained P450-6 mix mice F1 and F2 and genotyping of the fetus in the teratogenicity and fetal toxicity test, were carried out by PCR Fig. 5 shows the position of the primer in each gene and the chain length of the gene fragment generated by PCR Since the poly(A) addition signals in each gene are common, a reverse primer BGH-R is used in common, and a sequence specific to each gene is used as a forward primer. Based on the chain lengths of the gene fragments generated by the reaction, 1A1, 1B1, and COX-1 and, further 2E1, 3A7, and COX-2 can be simultaneously detected PCR was carried out in the following manner.

### (1) Sequences of primers

BGH-R: 5'-CTACTCAgACAATgCgATgC-3'
1A1-F: 5'-AgACCCTTATTgCTgTCCTg-3'
1B1-F2: 5'-AggTgCTTggAgTTTACCTg-3'
2E1-F2: 5'-AAACTCTgTgTCATTCCCCg-3'
3A7-F: 5'-ATCAgggATTCTgTACgTgC-3'
COX1-F: 5'-TgCTgAACTCCTTgTTAgCC-3'
COX2-F: 5'-CTCATTAgCCTgAATgTgCC-3'

### (2) Size of fragment to be amplified

1A1; about 520 bp
1B1; about 900 bp
2E1; about 380 bp
3A7; about 570 bp
COX1; about 730 bp
COX2; about 680 bp
1A1, 1B1, and COX1 and 2E1, 3A7, and COX2 can be simultaneously detected. Accordingly, typing is carried out by two PCR for each sample.

### (3) PCR conditions

1A1-F, 1B1-F2, and COX1-F and 2E1-F2, 3A7-F, and COX2-F are respectively adjusted at 10 pmol/µl. A reaction solution of 1 µl of genomic DNA, 2 µl of 10x PCR buffer, 1.6 µl of dNTP, 1 µl of BGH-R (10 pmol/µl), 1 µl of Primer-F mix (10 pmol/µl each), 0.1 µl of Ex Taq, and 13.3 µl of DDW (20 µl in total) was treated at 94°C for 5 minutes (hot start). PCR was then carried-out by repeating 30 times a cycle of 94°C for 30 seconds, 60°C for 30 seconds, and 72°C for 30 seconds. The solution was then treated at 72°C for 7 minutes, and was cooled to 4°C. After the completion of the reaction, 5 µl of the solution was run in 2% agarose gel.

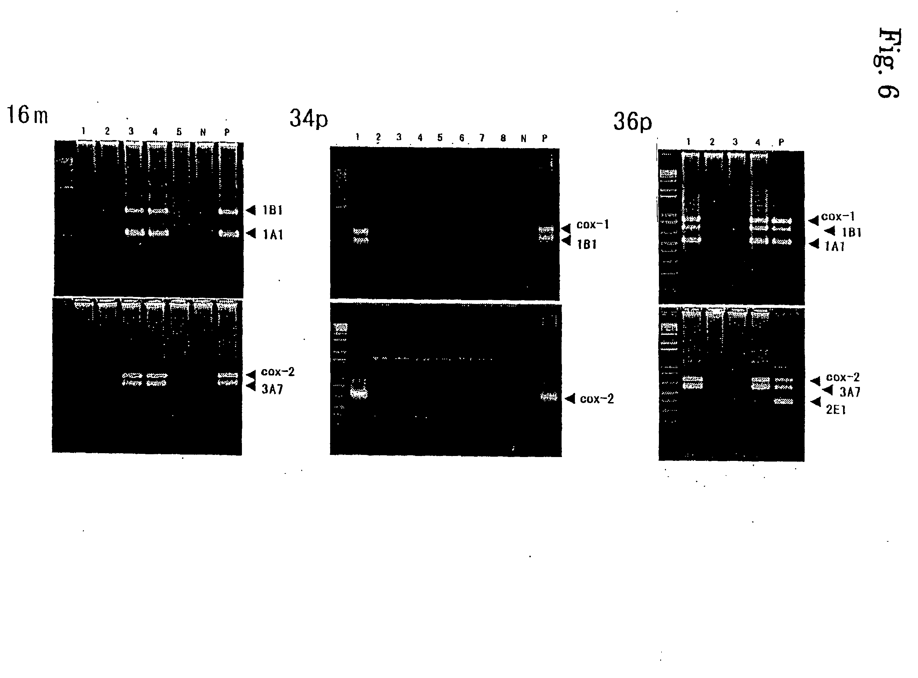

Fig. 6 shows the result of PCR for assaying the presence of the transgene in the transgenic mouse F1 produced in the Example. In Fig. 6, N represents a negative control, P represents a positive control, 16m represents 1A1, 1B1, 3A7, and COX-2 positive in individual 3 and individual 4, 34p represents 1B1, COX-1, and COX-2 positive in individual 1, and 36p represents 1A1, 1B1, 3A7, COX-1, and COX-2 positive in individual 1 and individual 4.

### Industrial Applicability

The use of the transgenic animal according to the present invention enables testing for forecasting fetal toxicity and teratogenicity of drugs.

## Claims

1. A recombinant gene comprising: (1) a gene encoding human P450 or a mutant gene thereof; (2) a human EF1 α promoter, (3) a chicken β-globin insulator sequence or a part thereof; and (4) an SV40 poly(A) addition signal.

2. A recombinant gene comprising: (1) a gene encoding human cyclooxygenase (PGH synthase) or a mutant gene thereof; (2) a human EF1 α promoter, (3) a chicken β -globin insulator sequence or a part thereof; and (4) an SV40 poly(A) addition signal.

3. The recombinant gene according to claim 1 which is capable of expressing human P450 or a mutant protein thereof in a non-human animal cell.

4. The recombinant gene according to claim 2 which is capable of expressing human cyclooxygenase or a mutant protein thereof in a non-human animal cell.

5. A recombinant vector which comprises the recombinant gene of claim 1 or 3.

6. A recombinant vector which comprises the recombinant gene of claim 2 or 4.

7. A transformant which comprises the recombinant vector of claim 5.

8. A transformant which comprises the recombinant vector of claim 6.

9. A transgenic non-human animal, a progeny thereof, or a part thereof, into which several genes which are selected from the human P450 gene or a mutant gene thereof and/or the human cyclooxygenase gene or a mutant gene thereof, have been introduced.

10. The transgenic non-human animal according to claim 9, a progeny thereof, or a part thereof, wherein the introduced human P450 gene or a mutant gene thereof and/or the human cyclooxygenase gene or a mutant gene thereof is expressed in the body of the animal.

11. The transgenic non-human animal according to claim 9 or 10, a progeny thereof, or a part thereof, wherein the introduced human P450 gene or a mutant gene thereof and/or the human cyclooxygenase gene or a mutant gene thereof is expressed in the body of a fetus.

12. The transgenic non-human animal according to any of claims 9 to 11, a progeny thereof, or a part thereof, wherein the human P450 gene or a mutant gene thereof is introduced as a recombinant gene comprising: (1) a gene encoding human P450 or a mutant gene thereof; (2) a human EF1 α promoter, (3) a chicken β-globin insulator sequence or a part thereof; and (4) an SV40 poly(A) addition signal.

13. The transgenic non-human animal according to any of claims 9 to 12, a progeny thereof, or a part thereof, wherein the human cyclooxygenase gene or a mutant gene thereof has been introduced as a recombinant gene comprising: (1) a gene encoding human cyclooxygenase (PGH synthase) or a mutant gene thereof; (2) a human EF1 α promoter, (3) a chicken β -globin insulator sequence or a part thereof; and (4) an SV40 poly(A) addition signal.

14. The transgenic non-human animal according to any of claims 9 to 13, a progeny thereof, or a part thereof, wherein the human P450 gene is the CYP1A1 gene, CYP1B1 gene, CYP2E1 gene, or CYP3A7 gene.

15. The transgenic non-human animal according to any of claims 9 to 14, a progeny thereof, or a part thereof, wherein the human cyclooxygenase gene is the cyclooxygenase-1 (COX-1) gene or cyclooxygenase-2 (COX-2) gene.

16. The transgenic non-human animal according to any of claims 9 to 15, a progeny thereof, or a part thereof, which is selected from the group consisting of:
a transgenic non-human animal into which the COX-1 gene and the COX-2 gene have been introduced;
a transgenic non-human animal into which the CYP1 gene, CYP1B1 gene, CYP3A7 gene, and COX-2 genes have been introduced;
a transgenic non-human animal into which the CYP1B1 gene, COX-1 gene, and the COX-2 gene have been introduced; and
a transgenic non-human animal into which the CYP1 gene, CYP1B1 gene, CYP3A7 gene, COX-1 gene, and COX-2 gene have been introduced

17. The transgenic non-human animal according to any of claims 9 to 16, a progeny thereof, or a part thereof, wherein the non-human animal is selected from the group consisting of mouse, rat, hamster, guinea pig, rabbit, dog, cat, horse, cattle, sheep, pig, goat, monkey, chicken, quail, vinegar fly, and nematode.

18. The transgenic non-human animal according to claim 17, a progeny thereof, or a part thereof, wherein the non-human animal is mouse.

19. A screening method for forecasting fetal toxicity and teratogenicity wherein the transgenic non-human animal of any of claims 9 to 18, a progeny thereof, or a past thereof is used.

20. The screening method according to claim 19 wherein a test compound is administered to the transgenic non-human animal of any of claims 9 to 18, a progeny thereof, or a part thereof, and the expression of fetal toxicity and teratogenicity is observed.

21. A substance which is judged to possess no fetal toxicity and no teratogenicity in the screening method of claim 19 or 20.

22. A pharmaceutical comprising the substance which is judged to possess no fetal toxicity and no teratogenicity in the screening method of claims 19 or 20.

23. The pharmaceutical according to claim 22 which is a preventive and therapeutic agent for the central nervous system disorders, psychiatric disorders, renal diseases, bone diseases, articular diseases, lung diseases, arteriosclerosis, heart diseases, diabetes, digestive system diseases, infectious diseases, allergic diseases, endocrine diseases, mental deterioration, or cancer.


SEQUENCE LISTING
<110> GenCom
<120> A transgenic animal having drug metabolic enzyme genes and its use
<130> A11598MA
<160> 21
<210> 1
<211> 20
<212> DNA
<213> Artificial Sequence
<220>
<223> Description of Artificial Sequence: Synthetic DNA
<400> 1
gggcaagcgg aagtgtatcg 20
<210> 2
<211> 20
<212> DNA
<213> Artificial Sequence
<220>
<223> Description of Artificial Sequence: Synthetic DNA
<400> 2
ggccacgcgt cgactagtac 20
<210> 3
<211> 20
<212> DNA
<213> Artificial Sequence
<220>
<223> Description of Artificial Sequence: Synthetic DNA

<400> 3

ccaacccaaa tgagcctgcg                                20

<210> 4

<211> 30

<212> DNA

<213> Artificial Sequence

<220>

<223> Description of Artificial Sequence: Synthetic DNA

<400> 4

gcgtcgacaa gattatgaaa gtgattttta                     30

<210> 5

<211> 20

<212> DNA

<213> Artificial Sequence

<220>

<223> Description of Artificial Sequence: Synthetic DNA

<400> 5

gtcctcccgg gctggcagca                                20

<210> 6

<211> 20

<212> DNA

<213> Artificial Sequence

<220>

<223> Description of Artificial Sequence: Synthetic DNA

<400> 6

ggccacgcgt cgactagtac                                20

<210> 7

<211> 29

<212> DNA

<213> Artificial Sequence

<220>

<223> Description of Artificial Sequence: Synthetic DNA

<400> 7

ccatgagccg gagtctcttg ctccggttc                    29

<210> 8

<211> 20

<212> DNA

<213> Artificial Sequence

<220>

<223> Description of Artificial Sequence: Synthetic DNA

<400> 8

tactccggag aacagatggg                    20

<210> 9

<211> 20

<212> DNA

<213> Artificial Sequence

<220>

<223> Description of Artificial Sequence: Synthetic DNA

<400> 9

gctccctttt ccctcaaggg                    20

<210> 10

<211> 30

<212> DNA

<213> Artificial Sequence

<220>

<223> Description of Artificial Sequence: Synthetic DNA

<400> 10

gcgtcgacta gaaagcgaat tttattagct 30

<210> 11

<211> 20

<212> DNA

<213> Artificial Sequence

<220>

<223> Description of Artificial Sequence: Synthetic DNA

<400> 11

tcagacagca aagcctaccc 20

<210> 12

<211> 21

<212> DNA

<213> Artificial Sequence

<220>

<223> Description of Artificial Sequence: Synthetic DNA

<400> 12

cttctacagt tcagtcgaac g 21

<210> 13

<211> 20

<212> DNA

<213> Artificial Sequence

<220>

<223> Description of Artificial Sequence: Synthetic DNA

<400> 13

gctccctttt ccctcaaggg 20
<210> 14
<211> 20
<212> DNA
<213> Artificial Sequence
<220>
<223> Description of Artificial Sequence: Synthetic DNA
<400> 14
gtttatcttc agaaaagatc tgtc 24
<210> 15
<211> 20
<212> DNA
<213> Artificial Sequence
<220>
<223> Description of Artificial Sequence: Synthetic DNA
<400> 15
ctactcagac aatgcgatgc 20
<210> 16
<211> 20
<212> DNA
<213> Artificial Sequence
<220>
<223> Description of Artificial Sequence: Synthetic DNA
<400> 16
agacccttat tgctgtcctg 20
<210> 17
<211> 20

<212> DNA
<213> Artificial Sequence
<220>
<223> Description of Artificial Sequence: Synthetic DNA
<400> 17
aggtgcttgg agtttacctg                                    20
<210> 18
<211> 20
<212> DNA
<213> Artificial Sequence
<220>
<223> Description of Artificial Sequence: Synthetic DNA
<400> 18
aaactctgtg tcattccccg                                    20
<210> 19
<211> 20
<212> DNA
<213> Artificial Sequence
<220>
<223> Description of Artificial Sequence: Synthetic DNA
<400> 19
   atcagggatt ctgtacgtgc                                    20
<210> 20
<211> 20
<212> DNA
<213> Artificial Sequence
<220>

<223> Description of Artificial Sequence: Synthetic DNA

<400> 20

tgctgaactc cttgttagcc                                    20

<210> 21

<211> 20

<212> DNA

<213> Artificial Sequence

<220>

<223> Description of Artificial Sequence: Synthetic DNA

<400> 21

ctcattagcc tgaatgtgcc                                    20